# EUROPEAN PATENT SPECIFICATION

(11) **EP 0 731 710 B1**
(45) Date of publication and mention of the grant of the patent: **14.05.2003**
(21) Application number: 94901265.2
(22) Date of filing: 05.11.1993
(51) Int. Cl.: A61K 38/22, A61P 31/12, A61P 43/00

(54) **THYMOSINE ALPHA 1 CONTAINING COMPOSITIONS FOR THE TREATMENT OF PATIENTS HAVING DECOMPENSATED LIVER DISEASE**
THYMOSIN-ALPHA-1 ENTHALTENDE ZUSAMMENSETZUNGEN ZUR BEHANDLUNG VON HEPATITIS B BEI PATIENTEN MIT DEKOMPENSIERTER LEBERERKRANKUNG
COMPOSITIONS CONTENANT DU THYMOSINE ALPHA 1 DESTINEES AU TRAITEMENT DE PATIENTS SOUFFRANT D'UNE MALADIE HEPATIQUE DECOMPENSEE

(43) Date of publication of application: 18.09.1996
(73) Proprietor: ALPHA 1 BIOMEDICALS, INC., Bethesda, MD 20817 (US); THE BOARD OF GOVERNORS OF WAYNE STATE UNIVERSITY, Detroit, MI 48202 (US)
(72) Inventor: CHRETIEN, Paul B., Rockville, MD 20852 (US); MUTCHNICK, Milton G., West Bloomfield, MI 48322 (US)
(74) Representative: Popp, Eugen, Dr.
(86) International application number: US9310619
(87) International publication number: WO95012405

(56) References cited:
- WO-A-93/05806
- WO-A-94/01125
- WO-A-95/11688
- US-A- 4 079 127
- MUTCHNICK M.G. ET AL.: "Thymosin treatment of chronic hepatitis B: a placebo-controlled pilot trial" HEPATOLOGY, vol. 14, no. 3, 1991, pages 409-415, XP002115980
- MUTCHNICK M.G. ET AL.: "Sustained response to thymosin therapy in patients with chronic hepatitis B" HEPATOLOGY, vol. 16, no. 4(2), 1992, page 66A XP002116413

## Description

### DESCRIPTION OF THE BACKGROUND ART

Hepatic decompensation is liver failure which can result from chronic or chronic active infection of a patient by Hepatitis B virus.

Of several known therapeutic agents which have been proposed for use in the treatment of Hepatitis B, the most extensively evaluated is interferon alpha-2b (hereinafter "α-interferon"), available commercially as INTRON® A. Unfortunately, the response rate of chronic Hepatitis B patients to α-interferon has been less than 50%. With the establishment of liver transplantation as a therapeutic modality for a variety of liver diseases, new expectations were raised for a cure of hepatic decompensation resulting from Hepatitis B infection. Unfortunately after some years of experience with liver transplantation, it is apparent that the rate of reoccurrence of Hepatitis B infection following transplantation is high.

In patients who have undergone liver transplantation wherein Hepatitis B virus DNA was detectable in the patient's serum prior to transplantation, the recurrence of Hepatitis B infection has been virtually universal within one year following transplantation. In view thereof, current medical practice precludes liver transplantation at many transplant centers in patients who have chronic Hepatitis B infection and who are serum positive for Hepatitis B virus DNA.

Treatment with α-interferon has not been successful in rendering serum of most patients with decompensated chronic Hepatitis B liver disease negative for Hepatitis B virus DNA. In fact, the INTRON® A label insert warns that α-interferon is contraindicated for patients exhibiting symptoms of hepatic failure, and may actually increase the risk of clinical decompensation. It is also known that α-interferon can lead to a level of decompensation which results in death.

In addition to α-interferon, another drug which has been suggested for treatment of Hepatitis B in patients is Thymosin α₁ ("Tα₁"). However, in view of the published warnings concerning the increased risk of hepatic decompensation when treating Hepatitis B using α-interferon, there would appear to be a negative motivation to use Thymosin α₁ in patients exhibiting symptoms of decompensated liver disease.

There remains a need in the art for methods of treating patients with hepatic decompensation so as to qualify such patients for liver transplantation.

### SUMMARY OF THE INVENTION

The invention provides a composition for use in treating a Hepatitis B patient having hepatic decompensation, comprising a pharmaceutical dosage unit containing a Hepatitis B virus-reducing amount of Tα₁, which pharmaceutical dosage unit can be administered to Hepatitis B-infected patient having decompensated liver disease, so as to render serum of said patient negative for Hepatitis B virus DNA.

The present invention is defined by claims 1 to 3 and relates to the use of pharmaceutical dosage units containing hepatitis B virus-reducing amount of Tα₁ for the manufacture of medicament for the treatment of hepatitis B in a patient having hepatic decomposition, each dosage unit comprising 1.6 mg Tα₁ or the pharmaceutical dosage unit of Tα₁ of 4mg.

### DESCRIPTION OF THE PREFERRED EMBODIMENTS

It has surprisingly been discovered that Thymosin α₁ ("Tα₁") can render Hepatitis B patients having decompensated liver disease serum negative for Hepatitis B virus DNA, thereby qualifying such patients for liver transplantation. This is surprising since the only approved drug for treatment of Hepatitis B, α-interferon, is contraindicated for use in patients with decompensated liver disease.

The terms "Thymosin α₁", "Thymosin alpha 1" and "Tα₁" as used herein encompass not only native (i.e., naturally occurring) Tα₁ but also synthetic Tα₁ and recombinant Tα₁ having the amino acid sequence of native Tα₁, amino acid sequences substantially similar thereto, or an abbreviated sequence from thereof, and their biologically active analogs (including muteins) having substituted, deleted, elongated, replaced, or otherwise modified sequences which possess bioactivity substantially similar to that of Tα₁.

Hepatitis B virus-reducing amounts of Thymosin α₁ are included within the dosage range of 0.4 - 4 mg.

Hepatitis B patients having decompensated liver disease are administered Thymosin α₁ until the patients are serum negative for Hepatitis B virus DNA, i.e., patients who become seronegative for Hepatitis B virus DNA in two consecutive monthly tests. A test for serum Hepatitis B virus DNA can be any suitable test, for example a radioimmunoassay, such as is available from Abbott Laboratories.

Thymosin α₁ is administered by subcutaneous injection twice weekly in pharmaceutical dosage units comprising 4 mg or 1.6 mg. However, it is to be understood that pharmaceutical dosage units containing Thymosin α₁ may be formulated in any suitable manner for administration by any suitable route. Suitable routes of administration may include parenteral (including subcutaneous, intramuscular, intravenous and intradermal), oral, and transdermal. Particularly preferred embodiments utilize parenteral administration.

Tα₁ can be administered in separate pharmaceutical dosage units. The pharmaceutical dosage units of the present invention include one or more pharmaceutically acceptable carriers and optionally other therapeutic ingredients. The carrier(s) are "acceptable" in the sense of being compatible with the other ingredients of the dosage unit formulation and not deleterious to the recipient thereof.

The pharmaceutical dosage unit formulations may be prepared by any suitable methods.

Use of the compositions may include the step of separately bringing into association the Tα₁ active ingredient with its carrier, which may comprise one or more ingredients. In general, the formulations are prepared by uniformly and intimately bringing into association the Tα₁ active ingredient with liquid carriers or finely divided solid carriers or both. Solid dosage unit formulations also may include the step of shaping the product.

Formulations of the present invention suitable for oral administration may be presented as discrete units such as capsules, cachets or tablets, containing a predetermined amount of the Tα₁ active ingredient; as a powder or granules; as a solution or a suspension in an aqueous liquid or a non-aqueous liquid; or as an oil-in-water liquid emulsion or a water-in-oil liquid emulsion, etc.

A tablet may be made by compression or molding, optionally with one or more accessory ingredients. Compressed tablets may be prepared by compressing, in a suitable machine, free-flowing powder or granules, optionally mixed with a binder, lubricant, inert diluent, preservative, surface-active or dispersing agent. Molded tablets may be made by molding, in a suitable machine, a mixture of the powdered compound moistened with an inert liquid diluent. Tablets may optionally be coated or scored and may be formulated so as to provide slow or controlled release of the active ingredient therein.

Formulations suitable for parenteral administration include aqueous and non-aqueous sterile injection solutions which may optionally contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze-dried (lyophilized) condition requiring only the addition of the sterile liquid carrier, for example water for injection, immediately prior to use. Extemporaneous injection solutions and suspensions may be prepared from sterile powders, granules and tablets of the kind previously described.

Suitable dosage units of Tα₁ can be administered to the patient daily, one or more times per day, e.g., two or three times per day, and doses can be administered one or more days per week, e.g., two, three, four, five, six or seven days per week.

After the patient becomes serum negative for Hepatitis B virus DNA, the decompensated liver of the patient can be removed, and a healthy liver then can be transplanted into the patient.

The invention is further illustrated by the following examples.

### Example 1

Patient 1 is a 48 year old physician who contracted hepatitis B surface antigen (HBsAg) positive hepatitis following a needle stick injury. Approximately 13 years later, liver biopsy showed chronic active hepatitis and cirrhosis. He suffered from significant fatigue. He had ascites and other manifestations of cirrhosis. Liver enzymes were elevated. Standard serologic studies were unusual in that Hepatitis B virus DNA, HBeAg, and HBsAg appeared negative. However, Hepatitis B virus DNA by the more sensitive PCR (polymerase chain reaction) assay was positive, as was Hepatitis B DNA testing of liver tissue obtained at biopsy. He was considered to have a mutant form of Hepatitis B virus infection. He failed to respond to α-interferon.

In an attempt to cure his Hepatitis B infection so that he would be eligible for transplant, he was started on Thymosin alpha 1 at a dose of 1.6 mg subcutaneously twice weekly. After approximately 3 months, he reported less fatigue. After roughly 6 months, Hepatitis B DNA PCR was negative. He had a liver transplant and returned to work shortly after his transplant. At his latest clinic visit, he was feeling well without elevations of his aminotransferase enzymes.

### Example 2

Patient 2 was a 69 year old man with very far-advanced cirrhosis secondary to long-standing chronic Hepatitis B infection. His findings included jaundice, weakness, severe encephalopathy, hepato-renal syndrome with renal failure, and portal gastropathy secondary to increased portal vein pressure. He received Thymosin alpha 1 at a dose of 1.6 mg subcutaneously twice weekly under a compassionate use protocol in an attempt to cure his Hepatitis B virus infection so that he could received a liver transplant. He tolerated therapy well, and had no side effects, but died from gastric bleeding, renal failure, and encephalopathy after 3 1/2 weeks of therapy. Death was associated with complications of portal hypertension and cirrhosis, and not due to therapy with Thymosin alpha 1.

### Example 3

Patient 3 was a 53 year old man with chronic Hepatitis B infection, cirrhosis, and esophageal varices. He also had a hepatoma, which recurred after a partial liver resection. Because of the limited amount of remaining normal hepatic tissue, another curative resection was not possible, and the patient's only hope for a cure was a transplant. However, he was not a candidate because of active infection with Hepatitis B.

He was started on Thymosin alpha 1 at a dose of 1.6 mg subcutaneously twice weekly, with the intention of treating him for 26 weeks. He tolerated therapy well and had no adverse effects. However, his therapy was discontinued after 46 injections because of complications of the cirrhosis. He expired about three weeks after his last injection. His death was attributed to bleeding esophageal varices and hepatoma. His hepatologist did not believe that Thymosin alpha 1 contributed to his death.

In summary, the above three patients with cirrhosis and hepatic decompensation received Thymosin alpha 1 at a dose of 1.6 mg subcutaneously twice weekly for periods varying from 3.5 weeks to 6 months. In each case, the intent of therapy was to cure chronic Hepatitis B virus infection so that the patient could be considered for transplant. None of the patients reported any adverse effects due to Tα₁. One of these patients had resolution of Hepatitis B virus infection while receiving Thymosin alpha 1, and later had a successful liver transplant, with no evidence of re-infection of the transplant by HBV. Two other patients, both of whom had far-advanced disease, died of complications of cirrhosis, with no evidence that Thymosin alpha 1 contributed to their deaths.

## Claims

1. The use of pharmaceutical dosage units containing hepatitis B virus-reducing amount of Tα₁ for the manufacture of medicament for the treatment of hepatitis B in a patient having hepatic decomposition, each dosage unit comprising 1.6 mg Tα₁.

2. The use of pharmaceutical dosage units containing hepatitis B virus-reducing amount of Tα₁ for the manufacture of medicament for the treatment of hepatitis B in a patient having hepatic decomposition, wherein the pharmaceutical dosage unit of Tα₁ is 4 mg.

3. The use of Claim 1, wherein said Tα₁ is present in an injectable or infusible pharmaceutical acceptable liquid carrier.

## Patentansprüche

1. Verwendung pharmazeutischer Dosiereinheiten, enthaltend eine Hepatitis B-Virus reduzierende Menge an Tα₁, zur Herstellung eines Medikaments zur Behandlung von Hepatitis B bei einem Patienten mit Leberzerfall, wobei jede Dosiereinheit 1,6 mg Tα₁ umfasst.

2. Verwendung pharmazeutischer Dosiereinheiten, enthaltend eine Hepatitis B-Virus reduzierende Menge an Tα₁, zur Herstellung eines Medikaments zur Behandlung von Hepatitis B bei einem Patienten mit Leberzerfall, wobei jede Dosiereinheit 4 mg Tα₁ umfasst.

3. Verwendung nach Anspruch 1, bei der Tα₁ in einem injizierbaren oder infundierbaren zulässigen pharmazeutischen flüssigen Träger vorhanden ist.

## Revendications

1. Application d'unités de doses pharmaceutiques contenant une quantité réductrice du virus de l'hépatite B de Tα₁ (thymosine α₁) à la fabrication d'un médicament pour le traitement de l'hépatite B d'un patient affecté par une décomposition hépatique, chaque unité de dose contenant 1,6 mg de Tα₁.

2. Application d'unités de doses pharmaceutiques contenant une quantité réductrice du virus de l'hépatite B de Tα₁ (thymosine α₁) à la fabrication d'un médicament pour le traitement de l'hépatite B d'un patient affecté par une décomposition hépatique, dans laquelle l'unité de dose de Tα₁ est de 4 mg.

3. Application selon la revendication 1, dans laquelle Tα₁ est présent dans un véhicule liquide acceptable pharmaceutique pour injection ou infusion.
